# EUROPÄISCHE PATENTSCHRIFT

(11) **EP 0 808 615 B1**
(45) Veröffentlichungstag und Bekanntmachung des Hinweises auf die Patenterteilung: **10.07.2002**
(21) Anmeldenummer: 97106732.7
(22) Anmeldetag: 23.04.1997
(51) Int. Cl.: A61F 2/52

(54) **Brustprothese und Verfahren für deren Herstellung**
Breast prosthesis and method for the manufacture thereof
Prothèse mammaire et procédé pour sa fabrication

(30) Priorität: 23.04.1996 DE 19616190; 15.01.1997 DE 29700642 U; 13.03.1997 DE 29704605 U
(43) Veröffentlichungstag der Anmeldung: 26.11.1997
(73) Patentinhaber: F + E Gesellschaft für Bekleidungsinnovation mbH & Co. KG, 83098 Brannenburg (DE)
(72) Erfinder: Weber-Unger, Georg, 6330 Kufstein (AT); Volk, Stephan, 83714 Miesbach (DE)
(74) Vertreter: Haug, Dietmar, Dipl.-Ing.

(56) Entgegenhaltungen:
- WO-A-94/16650
- DE-A- 4 421 516
- US-A- 4 125 117

## Beschreibung

### Technisches Gebiet

Die Erfindung betrifft eine Brustprothese mit einem weichelastischen Kunststoffkörper, der zwei längs ihres gemeinsamen Randes miteinander verbundene Kunststoffolien und eine zwischen den Kunststoffolien eingeschlossene Kunststoffmasse aufweist, wobei der Kunststoffkörper eine der Form der natürlichen Brust nachgebildete Vorderseite und eine dem Oberkörper der Trägerin zuwendbare Rückseite hat, und einem elastisch verformbaren, flächigen Teil, das an seinem Rand an der Rückseite des Kunststoffkörpers befestigt ist. Die Erfindung betrifft auch ein Verfahren für die Herstellung der Brustprothese. Derartige Brustprothesen werden in Büstenhaltern, Bikinioberteilen, Badeanzügen oder ähnlichem getragen.

### Stand der Technik

Die DE 44 21 516 C1 offenbart eine Brustprothese gemäß dem Oberbegriff von Anspruch 1 und ein Verfahren zur Herstellung von einer Brustprothese gemäß dem Oberbegriff von Anspruch 31.

Die aus der DE 44 21 516 C1 bekannte Brustprothese stellt eine äußerst angenehm zu tragende und natürlich wirkende Brustprothese zur Verfügung. Da Brustprothesen sehr oft lange Zeit getragen werden sowie ein für das Selbstwertgefühl der Frau wichtiges Körperteil nachbilden, stellen selbst geringfügige Verbesserungen des Tragekomforts bzw. der Simulation des natürlichen Eindrucks einen großen Fortschritt dar.

### Darstellung der Erfindung

Die Aufgabe der Erfindung besteht darin, die gattungsgemäße Brustprothese so auszubilden, daß der Tragekomfort bzw. die Simulation des natürlichen Eindrucks weiter verbessert werden, sowie ein Verfahren für deren Herstellung anzugeben.

Die Aufgabe wird mit den Merkmalen des Anspruchs 1 bzw. 31 gelöst. Vorteilhafte Ausgestaltungen sind in den abhängigen Ansprüchen offenbart.

Da die Prothese im Randbereich vorzugsweise nur die die Kunststoffmasse einschließenden Kunststoffolien aufweist, läßt sich ein nahezu stufenloser oder natürlicher Übergang zwischen der Prothese und der Haut der Prothesenträgerin erreichen. Dieser Vorteil der erfindungsgemäßen Ausgestaltung wird besonders bei Verwendung eines Stoffteils für den flächigen Teil deutlich, weil die Materialstärke des Stoffteils einen abgestuften Übergang bedingt.

Einige Trägerinnen bevorzugen es, die Brustprothese direkt auf der Haut zu tragen. Durch die erfindungsgemäße Ausgestaltung wird das Risiko des Verrutschens der Prothese vermindert, was zu einem unnatürlichen und somit unerwünschten Eindruck führen könnte. Der Vorteil beruht darauf, daß der nicht durch das flächige Teil, das vorzugsweise ein Stoffteil ist, abgedeckte Randbereich der Prothese, auf dem die Prothese auf der Haut der Trägerin aufliegt, einen höheren Haftreibungskoeffizienten als das flächige Teil aufweist. Dieser Effekt wird durch die natürliche Feuchtigkeit der Haut verstärkt.

Bei Teilamputationen oder nach dem Wiederaufbau einer neuen, aber kleineren Brust bei einer Vollamputation werden vorzugsweise Schalenprothesen verwendet, die lediglich einen Teil der natürlichen Brust nachbilden. Um einen möglichst natürlichen oder stufenlosen Übergang zwischen der verbliebenen bzw. wieder aufgebauten Brust und der Prothese zu schaffen, nimmt die Stärke des Kunststoffkörpers derartiger Schalenprothesen bis zum Rand allmählich ab. Daher ist die Dicke und damit Festigkeit des weichelastischen Kunststoffkörpers gegenüber Vollprothesen vermindert. Erfindungsgemäß wird die Gefahr des Einrollens des Randes des Kunststoffkörpers infolge der Einwirkung einer aus der Spannung des flächigen Teils resultierenden Kraft vermieden, da das flächige Teil nach innen versetzt angeordnet ist. Deshalb wird der Kunststoffkörper allenfalls eingedellt, aber auf keinen Fall eingerollt. Zudem wird diese Verformung durch die größere Wandstärke des Kunststoffkörpers und die damit einhergehende erhöhte Steifigkeit verringert. Vorzugsweise ist das flächige Teil 1 bis 4 cm innerhalb des Randes der Brustprothese an der Rückseite des Kunststoffkörpers befestigt. Der Abstand variiert vorzugsweise sowohl mit der Größe der nachzubildenden Brust als auch mit der Stelle der Befestigung.

Vorzugsweise ist in dem flächigen Teil und/oder zwischen dem flächigen Teil und dem Kunststoffkörper eine Öffnung vorgesehen, um Füllmaterial zwischen das flächige Teil und die Rückseite der Brustprothese einzubringen. Dadurch kann die Prothese individuell an den Oberkörper, d.h. die verbliebene bzw. wieder aufgebaute Brust angepaßt werden. Insbesondere können Veränderungen berücksichtigt werden. Auch kann die Trägerin die Anordnung des Füllmaterials für einen besseren Tragekomfort und Sitz der Prothese selbst durch Probieren anpassen.

Vorzugsweise ist das flächige Teil derart tiefgezogen, daß er an die konkave Form der Rückseite des Kunststoffkörpers spannungsfrei angepaßt ist, wodurch sich der Vorteil ergibt, daß es auch spannungsfrei an dem nach einer Teilamputation verbleibenden oder vollständig erhaltenen Brustgewebe anliegt, wodurch der Tragekomfort erhöht wird.

Ein weiterer Vorteil der erfindungsgemäßen Brustprothese besteht darin, daß sie als Ausgleichsschale sowohl bei einer Reduktion als auch zur Vergrößerung einer Brust verwendet werden kann. Außerdem kann sie bei einem stufenweisen Wiederaufbau der Brust an das sich ändernde Volumen und Gewicht der Brust durch eine entsprechende stufenweise Verringerung des Füllmaterials leicht angepaßt werden.

Bei der erfindungsgemäßen Brustprothese ist das flächige Teil innerhalb des gemeinsamen Randes der miteinander verbundenen Kunststoffolien an dem Kunststoffkörper befestigt. Dadurch ist das flächige Teil näher an der Brustvorderseite oder anders ausgedrückt weiter innerhalb der Brustprothese angeordnet. Ein weiterer Vorteil ergibt sich, wenn ein Material für das flächige Teil gewählt wird, das beim Tiefziehen nicht oder nur wenig gemoldet wird, sondern derart elastisch ist, daß das flächige Teil seinen Flächeninhalt, den es vor dem Tiefziehen hatte, im wesentlichen wieder einnimmt. Dadurch entsteht eine lose gespannte Fläche. In erster Näherung kann dann die erfindungsgemäße Brustprothese als ein elastischer Hohlkörper angesehen werden. Wird auf einen elastischen Hohlkörper derart eine Kraft ausgeübt, daß er in einer Richtung gestaucht wird, vergrößert sich die Querschnittsfläche zwischen den Begrenzungsflächen. Diese Vergrößerung ist beispielsweise bei einem Zylinder oder einer Kugel in der Mitte am größten. Verbindet man in der Querschnittsfläche die Hülle des Hohlkörpers, muß bei der Vergrößerung der Querschnittsfläche auch das Verbindungsmittel verformt werden. Die dafür aufzuwendende Kraft wirkt der Stauchkraft entgegen. Der Körper wird zusätzlich zu der der Hülle eigenen Steifigkeit durch ein der Querschnittsflächenvergrößerung entgegenwirkendes Verbindungsmittel versteift.

Bei einer derartigen erfindungsgemäßen Brustprothese wird der Kunststoffkörper durch das flächige Teil an seiner Befestigungslinie zusätzlich zu der ihm eigenen Steifigkeit versteift. Bei gleicher Steifigkeit kann die Brustprothese deshalb leichter ausgestaltet werden, wodurch der Tragekomfort gesteigert wird. Außerdem ist die Brustprothese kostengünstiger herzustellen. Je nach Bedarf und Größe der nachzubildenden Brust können ein, zwei oder mehrere flächige Teile mit dem Kunststoffkörper verbunden werden. Durch die geeignete Auswahl der Zahl und Anordnung der flächigen Teile kann die Brustprothese an das Verhalten der natürlichen Brust optimal angepaßt werden. Zufriedenstellende Ergebnisse werden mit einem ersten flächigen Teil im hinteren Drittel und einem zweiten flächigen Teil im vorderen Drittel der Brustprothese erreicht.

Um den Tragekomfort der Brustprothese für diejenigen Frauen, die keinen direkten Kontakt mit der Kunststoffolie der Brustprothese mit ihrer Haut wünschen, zu erhöhen, wird vorzugsweise das flächige Teil, das dem Oberkörper der Trägerin am nächsten liegt, bis zum gemeinsamen Rand der Kunststoffolien um einen Randbereich erweitert, der vorzugsweise Microfaserstoff enthält.

Vorteilhafterweise erfolgt die Verbindung des abschließenden Rands des Randbereichs an dem Kunststoffkörper mit der gleichen Technik wie die Verbindung der beiden Kunststoffolien. Das kann beispielsweise durch Verschweißen oder Verkleben erfolgen. Die feste Verbindung zwischen dem abschließenden Rand des Randbereichs des flächigen Teils und dem Kunststoffkörper ermöglicht es, den abschließenden Rand genau bündig mit dem Rand der Kunststoffolien zu machen. Dadurch wird ein überstehender Rand vermieden, der aus dem Bekleidungsstück herausragen könnte, in dem die Brustprothese getragen wird.

Vorteilhafterweise ist der Randbereich des flächigen Teils rasterähnlich mit der dem Oberkörper der Trägerin zuwendbaren Rückseite fest verbunden. Das kann beispielsweise durch Verschweißen mittels eines rasterförmigen Werkzeugs geschehen. Dadurch wird ein sicherer und rutschfester Sitz des flächigen Teils und damit der Brustprothese erreicht. Daher wird die Sicherheit gegen ein Verrutschen bzw. eine unnatürliche Wirkung der Brustprothese erhöht.

Die Brustprothese berührt nur im Randbereich den Oberkörper der Trägerin. Die empfindliche Haut und das Narbengewebe werden in starkem Umfang belüftet und beschleunigen bei einer frischamputierten Trägerin den Vernarbungsprozeß bzw. sorgen für ein hautfreundliches Klima. Durch die ständige Bewegung am Körper kommt es zu einem Saug- und Pumpeffekt, der einen Luftaustausch zwischen der Haut und der Prothese gewährleistet.

Durch die Anordnung des flächigen Teils innerhalb des Randes des Kunststoffkörpers entsteht eine Vertiefung, in die bei einer Teilamputation stehengebliebenes Brustgewebe aufgenommen werden kann, ohne daß ein störender Druckkontakt zwischen dem flächigen Teil und dem restlichen Brustgewebe entsteht. Wenn bei einer Teilamputation hingegen mehr Brustgewebe erhalten werden kann, als die Vertiefung ohne Berührung zwischen Brustgewebe und flächigem Teil aufnehmen kann, drückt das verbleibende Brustgewebe weniger stark auf das flächige Teil als bei einer Brustprothese, bei der das flächige Teil am Rand des Kunststoffkörpers befestigt ist.

Vorzugsweise besteht das flächige Teil aus einem Microfaseroder Klimastoff. Weil Schweiß in Dampfform durch ein solches Material hindurchtreten kann und nach Kondensation an der Rückkehr zur Haut der Trägerin gehindert wird, bleibt die Haut auch bei stärkerer körperlicher Anstrengung immer angenehm trocken.

Anstatt das flächige Teil durch Schweißen mit dem Kunststoffkörper zu verbinden, kann das flächige Teil mit dem Kunststoffkörper auch verklebt werden. Auch das Kleben führt zu einer festen und dauerhaften Verbindung zwischen den Teilen. Die Befestigung durch Kleben ist immer dann angebracht, wenn sich das für das flächige Teil gewählte Material nicht oder weniger gut zum Schweißen eignet, beispielsweise wenn es ganz oder teilweise aus einer Naturfaser wie Baumwolle besteht.

Das Verschweißen des flächigen Teils mit dem Kunststoffkörper bedeutet nicht zwangsläufig, daß das flächige Teil mit dem Kunststoffkörper vollständig verschmilzt. Unter dem Begriff "Verschweißen" soll in dieser Beschreibung auch ein Befestigungsverfahren verstanden werden, bei dem das flächige Teil nur an seiner Oberfläche mit der angrenzenden Kunststoffolie des Kunststoffkörpers verschmilzt.

Für das flächige Teil kann eine Vielzahl verschiedener Materialien verwendet werden. Die wesentlichen Kriterien bei der Auswahl des für das flächige Teil zu verwendenden Materials sind seine Fähigkeit, leicht an dem Kunststoffkörper befestigt und evtl. leicht davon wieder gelöst werden zu können, eine erwünschte Dehnbarkeit bzw. Steifigkeit, eine hohe Reißfestigkeit, ein gutes Feuchtigkeitsaufnahme- und -abgabevermögen, eine angenehme Weichheit und die Fähigkeit, leicht gereinigt werden zu können. Wenn das flächige Teil an den Kunststoffkörper angeschweißt werden soll, ist es von Vorteil, wenn es aus Polyamid- oder Polyesterfasern hergestellt ist. Wegen seiner bekannten hautfreundlichen Eigenschaften kann das flächige Teil mindestens in dem Randbereich aus Baumwolle oder aus einer Mischung aus Polyamid- und/oder Polyesterfasern und Baumwolle bestehen. Soll das flächige Teil besonders gut Feuchtigkeit von der Hautoberfläche abtransportieren, ist es von Vorteil, wenn es ein Gewebe oder Gewirk aus Microfasern ist, das für Feuchtigkeit in Dampfform durchlässig ist, aber kondensierte Feuchtigkeit am Durchtritt hindert. Besonders geeignet sind feinstfibrillige Microfasern, die aus Polyester oder Polyamid bestehen. Derartige Microfaser-Gewebe sind dem Fachmann unter der Bezeichnung "Klimastoffe" bekannt. Je nach den verschiedenen Anforderungen können die verschiedenen Abschnitte unterschiedliche Materialien aufweisen. Beispielsweise weisen insbesondere die vorderen flächigen Teile vorzugsweise eine Kunststoffolie auf, die z.B. aus Polyurethan ist.

Bevorzugt ist in der Brustprothese eine Öffnung vorgesehen, um Füllmaterial in den entstandenen Hohlraum zwischen der oder dem gespannten Flächen und der dem Oberkörper der Trägerin zuwendbaren Rückseite einbringen zu können. Die Öffnung ist vorzugsweise wieder verschließbar, insbesondere mittels eines Klettverschlusses. Sie kann in der Schweißnaht zwischen dem flächigen Teil und der dem Oberkörper der Trägerin zuwendbaren Rückseite oder in dem flächigen Teil selbst vorgesehen sein. Das Füllmaterial ist vorzugsweise ein Polsterkörper, insbesondere ein Microfaser-Garnbausch oder ein luftdichter Folienball oder ein Schaumstoffkörper. Die Öffnung bietet den Vorteil, daß der Polsterkörper entnehmbar oder ergänzbar ist.

Vorzugsweise weist die Öffnung zum Befüllen der Brustprothese eine Einrichtung zum Verstärken der Stoffränder auf. Dazu ist eine Kunststoffolie zwischen dem Stoffteil und dem Stoffband angeordnet und mit dem Stoffteil und dem Stoffband verschweißt oder verklebt. Dadurch wird im Randbereich des Stoffteiles ein aus Stoff teil, Stoffband und Kunststoffolie bestehendes Laminat geschaffen, das den Rand des Stoffteiles und auch den des Stoffbandes zuverlässig vor dem Ausfransen schützt, das wirtschaftlich herstellbar ist, das nicht oder nur unwesentlich dicker als das Stoffteil und das Stoffband ist, wenn beide aufeinander liegen, und das weder das Aussehen des Stoffteiles noch das Aussehen des Stoffbandes verändert.

Vorzugsweise ist die Kunststoffolie bzw. sind die Kunststofffolie Polyurethanfolien, weil diese sehr dünn, hochelastisch und transparent sein kann und gut mit dem Stoffteil und dem Stoffband verschweißt werden können.

Ferner können das Stoffteil und das Stoffband aus dem gleichen Material, vorzugsweise einem Stoff aus Polyamid- oder Polyesterfasern, sein, wodurch die Haftung zwischen der Kunststofffolie und dem Stoffteil und die Haftung zwischen der Kunststoffolie und dem Stoffband gleich groß ist.

### Kurze Beschreibung der Zeichnungen

Verschiedene Ausführungsbeispiele der Erfindung werden in den Zeichnungen dargestellt und im folgenden näher beschrieben. Die Dicken der Kunststoffolien und des Stoffteils sind dabei im Verhältnis zu den Abmessungen der Brustprothese übertrieben dick dargestellt.

Es zeigen:
Figur 1 einen Schnitt durch eine erfindungsgemäße Brustprothese;
Figur 2 einen Schnitt durch die Brustprothese von Fig. 1, wobei Füllmaterial zwischen das flächigen Teil und die Rückseite des Kunststoffkörpers eingebracht ist;
Figuren 3 bis 5 ein Herstellungsverfahren für eine erfindungsgemäße Brustprothese, wobei die Brustprothese bei einzelnen Fertigungsschritten im Schnitt dargestellt ist;
Figur 6 einen Querschnitt durch eine erfindungsgemäße Brustprothese gemäß einem weiteren Ausführungsbeispiel;
Figur 7 einen Querschnitt durch ein weiteres Ausführungsbeispiel einer erfindungsgemäßen Brustprothese, bei der der flächige Teil einen Randbereich aufweist;
Figur 8 einen Querschnitt durch ein weiteres Ausführungsbeispiel einer erfindungsgemäßen Brustprothese, bei der mehrere flächige Teile vorgesehen sind;
Figuren 9A, 9B und 9C Querschnitte durch weitere Ausführungsbeispiele erfindungsgemäßer Brustprothesen, wobei ein Polsterkörper in einem Hohlraum zwischen Kunststoffkörper und flächigem Teil vorgesehen ist; und
Figuren 10A, 10B und 10C Querschnitte durch weitere Ausführungsbeispiele erfindungsgemäßer Brustprothesen, wobei eine Öffnung zur Entnahme oder Ergänzung des Polsterkörpers vorgesehen ist;
Figur 11 eine Draufsicht auf einen Ausschnitt eines Stoffteiles, in dem sich eine schlitzförmige Öffnung befindet, deren Rand erfindungsgemäß verstärkt ist; und
Figur 12 einen Schnitt durch das in Fig. 11 dargestellte Stoffteil längs der Linie XII-XII, wobei zusätzlich zu dem Stoffteil das Stoffband und die Kunststofffolie zu sehen sind.

### Bevorzugte Ausführungsbeispiele der Erfinfdung

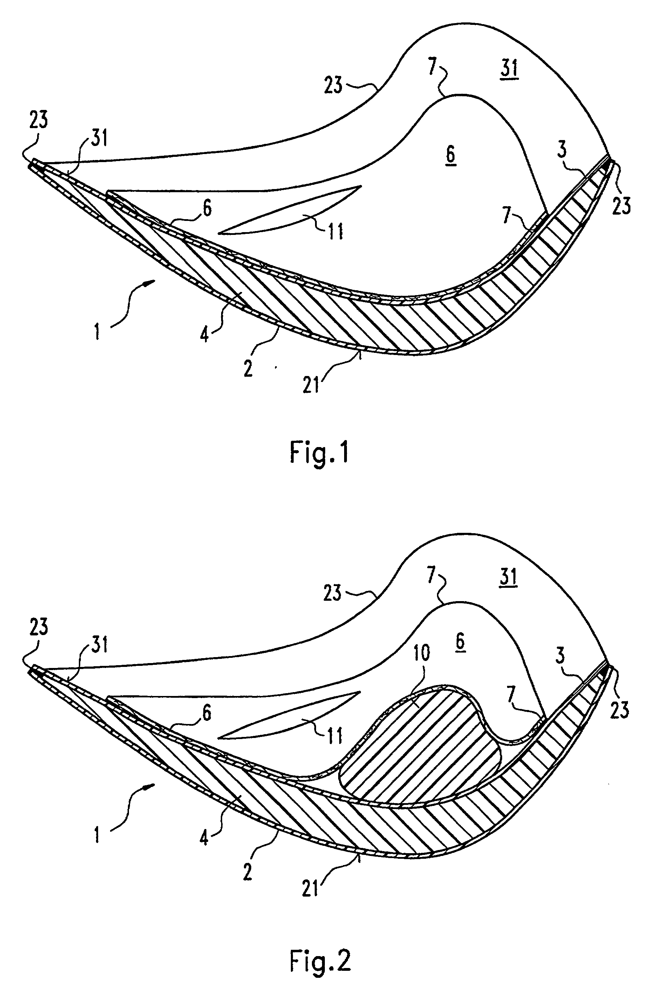

Die in Figur 1 dargestellte Brustprothese stellt die bevorzugte Ausführungsform der Erfindung dar. Die Brustprothese weist einen weichelastischen, schalenförmigen Kunststoffkörper 1 auf, der aus zwei längs ihres gemeinsamen Randes 23 miteinander verschweißten Polyurethanfolien 2, 3 und einer zwischen die Polyurethanfolien hohlraumfrei eingeschlossenen additionsvernetzten Zwei-Komponenten-Silikon-Kautschukmasse 4 besteht. Die der Silikonmasse 4 abgewandte Seite 21 der Polyurethanfolie 2 hat eine konvexe Form, die der Form der natürlichen Brust entspricht. Die Seite 21 der Polyurethanfolie 2 stellt die Vorderseite des Kunststoffkörpers 1 und damit die Vorderseite der Brustprothese dar. Die der Silikonmasse 4 gegenüberliegende Seite 31 der Polyurethanfolie 3 hat eine konkave Form, die einen der Vorderseite des Kunststoffkörpers annähernd entsprechenden Verlauf hat. Diese Seite 31 der Polyurethanfolie 3 stellt die dem Körper der Prothesenträgerin zugekehrte Rückseite des Kunststoffkörpers 1 dar. Mit der Rückseite des Kunststoffkörpers 1 ist ein elastisch verformbares Stoffteil (flächiges Teil) 6 durch mindestens eine Schweiß- oder Klebenaht 7 fest verbunden. Die mindestens eine Schweiß- oder Klebenaht 7 verläuft innerhalb des gemeinsamen Rands 23 der beiden Kunststoffolien 2,3. Sie kann ununterbrochen sein oder aus Teilnähten bestehen, zwischen denen in Längsrichtung ein oder mehrere Abstände sind, der bzw. die als Füllöffnung für Füllmaterial dienen kann oder können.

Das dargestellte Ausführungsbeispiel stellt eine Schalenprothese dar, die eine verbleibende oder wieder aufgebaute Brust symmetrisch zu der erhaltenen Brust ergänzt. Da jeder Mensch nicht vollkommen symmetrisch ist, kann die nachgebildete Brust je nach Wunsch der Trägerin auch etwas größer oder kleiner nachgebildet sein.

Zum Rand des Kunststoffkörpers hin läuft die Schalenprothese allmählich flach aus, um einen möglichst stufenlosen Übergang zwischen der Haut der Trägerin und der Prothese zu schaffen.

Die Rückseite 31 des Kunststoffkörpers zwischen dem gemeinsamen Rand 23 der beiden Kunststoffolien 2, 3 und dem Stoffteil 6 ist vorzugsweise nicht von einem Stoffteil bedeckt. Das bedeutet eine größere Haftreibung, wenn die Prothese direkt auf der Haut getragen wird. Dadurch wird die Gefahr des Verrutschens der Prothese und die damit einhergehende Entstellung der Trägerin verringert.

Zwischen das Stoffteil 6 und die von ihm bedeckte Rückseite 31 des Kunststoffkörpers 1 kann Füllmaterial eingebracht werden, um die Schalenprothese optimal an die verbliebene bzw. wieder aufgebaute Brust anzupassen. Fig. 2 zeigt die Brustprothese von Fig. 1, wobei zwischen das Stoffteil 6 und den Kunststoffkörper 1 Füllmaterial 10 eingebracht ist.

Das Stoffteil 6 weist vorzugsweise eine schlitzförmige Öffnung 11 auf, um das Füllmaterial zwischen das Stoffteil und die Rückseite 3 des Kunststoffkörpers einbringen bzw. entfernen zu können. Vorzugsweise ist auf der dem Kunststoffkörper zugewandten Seite des Stoffteils 6 ein ringförmiges Stoffband derart angeordnet, daß die die Öffnung 11 begrenzenden Ränder des Stoffteils 6 und des Stoffbandes bündig aufeinander liegen.

Vorzugsweise befindet sich zwischen dem Stoffteil und dem Stoffband eine Kunststoffolie, welche den gleichen Umriß wie das Stoffband hat und deckungsgleich mit dem Stoffband angeordent ist. Die Kunststoffolie ist vollflächig sowohl mit dem Stoffband als auch mit dem Stoffteil 6 verschweißt. Durch den Einsatz des Stoffbandes und der Kunststoffolie ist es möglich, die Ränder der Öffnung 11 ohne Verwendung einer Naht zu verstärken.

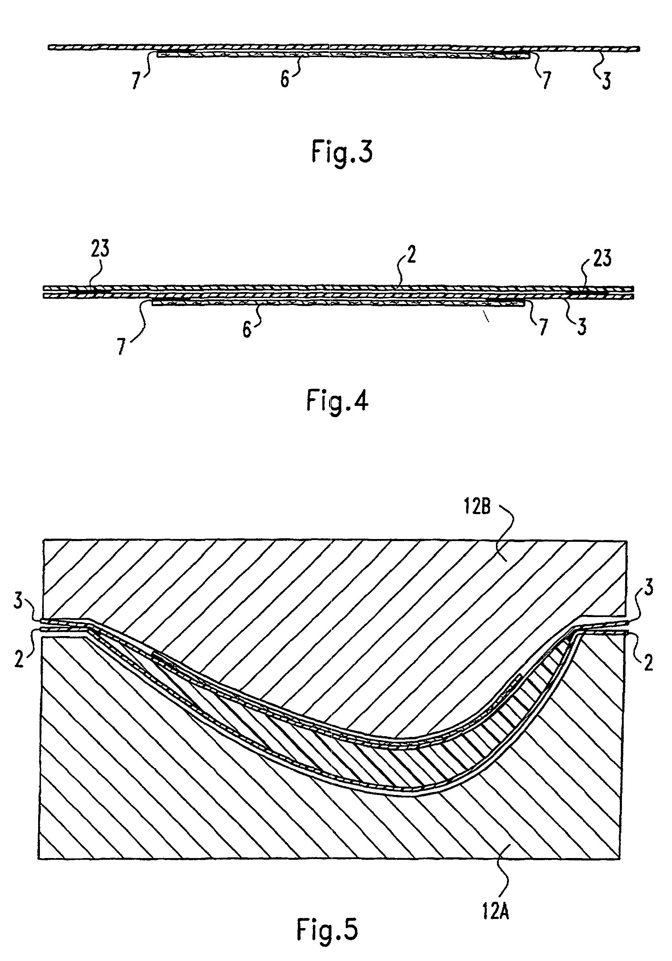

Im folgenden wird das bevorzugte Verfahren zur Herstellung einer Brustprothese anhand der Figuren 3 bis 5 erläutert:
(A) Zunächst wird das Stoffteil 6 zugeschnitten und gegebenenfalls mit einer Öffnung 11 versehen. Vorzugsweise wird der Rand der Öffnung beispielsweise durch Verschweißen mit einer Kunststoffolie und einem Stoffband verstärkt.
(B) Im nächsten Schritt wird das vorbereitete Stoffteil 6 an einer Kunststoffolie 3 vorzugsweise durch Schweißen befestigt. Dieser Zustand ist in Fig. 3 dargestellt. Vorzugsweise ist das verstärkende Stoffband zwischen der Kunststoffolie 3 und dem Stoffteil 6 angeordnet.
(C) Im Anschluß wird eine weitere Kunststoffolie 2 auf die dem Stoffteil 6 gegenüberliegende Seite der Kunststoffolie 3 gelegt und längs einer den späteren Prothesenrand bildenden Linie 23 bis auf eine später zu schließende Einfüllöffnung miteinander derart vorzugsweise durch Verschweißen verbunden, daß ein Beutel entsteht. Die den späteren Prothesenrand bildende Linie 23 umgibt den Rand 7 des Stoffteils 6. Dieser Verfahrenszustand ist in Fig. 4 gezeigt.
(D) Der auf diese Weise hergestellte Beutel wird dann mit unvernetzter Silikonkautschukmasse befüllt und in einer zweiteiligen Form 12A, 12B angeordnet, die eine Aushöhlung aufweist, deren Form der der natürlichen Brust nachgebildeten Vorderseite der Prothese entspricht. Auf bekannte Weise wird die Form 12A, 12B geschlossen. Dabei werden die beiden Kunststoffolien 2, 3 und das Stoffteil 6 tiefgezogen. Das Stoffteil 6 weist vorzugsweise synthetisches Material derart auf, daß das Stoffteil 6 bei dem Tiefziehverfahren gemoldet wird und vorzugsweise eine der tiefgezogenen Rückseite 31 entsprechende vorgeformte Gestalt annimmt. Alternativ kann das Material seine ursprüngliche Form nach dem Tiefziehen beibehalten, wodurch die Brustprothese durch die dadurch entstandende lose gespannte Fläche versteift wird. Die tiefgezogene Brustprothese in der Form ist in Fig. 5 gezeigt. Der Übersicht halber sind die den Figuren 1 und 2 entsprechenden Bezugszeichen in Fig. 5 weggelassen.
(E) In einem Wärmeschrank wird anschließend die Vernetzung der Silikonkautschukmasse bewirkt. Danach wird die Prothese aus der Form 12A, 12B entfernt und der Endbearbeitung zugeführt, wo insbesondere der noch überstehende Randbereich der Kunststoffolien mit bis zu der Verbindungslinie 23 abgeschnitten, die dann den Rand der fertigen Prothese darstellt.

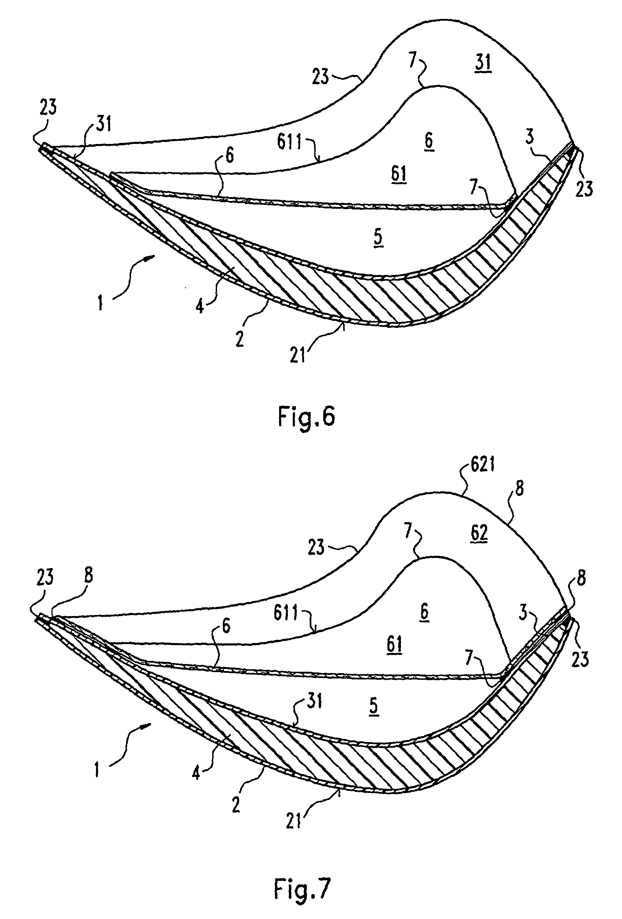

Die in Figur 6 dargestellte Brustprothese weist einen weichelastischen, schalenförmigen Kunststoffkörper 1 auf, der aus zwei längs ihres gemeinsamen Randes 23 miteinander verschweißten Polyurethanfolien 2, 3 und einer zwischen die Polyurethanfolien hohlraumfrei eingeschlossenen additionsvernetzten Zwei-Komponenten-Silikon-Kautschukmasse 4 besteht. Die der Silikonmasse 4 abgewandte Seite 21 der Polyurethanfolie 2 hat eine konvexe Form, die der Form der natürlichen Brust entspricht. Diese Seite 21 der Polyurethanfolie 2 stellt die Vorderseite des Kunststoffkörpers 1 und damit die Vorderseite der Brustprothese dar. Die der Silikonmasse 4 gegenüberliegende Seite 31 der Polyurethanfolie 3 hat eine konkave Form, die einen der Vorderseite des Kunststoffkörpers annähernd entsprechenden Verlauf hat. Diese Seite 31 der Polyurethanfolie 3 stellt die dem Körper der Prothesenträgerin zugekehrte Rückseite des Kunststoffkörpers 1 dar. Mit der Rückseite des Kunststoffkörpers 1 ist ein elastisch verformbares Stoffteil (flächiges Teil) 6 durch eine Schweiß- oder Klebenaht 7 fest verbunden. Die Schweiß- oder Klebenaht 7 verläuft innerhalb des gemeinsamen Rands 23 der beiden Kunststoffolien 2,3. Daher bildet das Stoffteil 6 eine Fläche 61, die dichter in bezug auf die Prothesenvorderseite angeordnet ist, als bei einer Ausführung einer Brustprothese, bei der das Stoffteil nur an dem gemeinsamen Rand 23 der beiden Kunststoffolien 2, 3 befestigt ist. Der Flächeninhalt der Fläche 61 würde sich bei Druckeinwirkung und damit einhergehender Formveränderung der Brustprothese vergrößern. Die dazu notwendige Kraft erzeugt eine der Deformation entgegenwirkende Kraft. Da die Fläche 61 dichter an der Prothesenvorderseite angeordnet ist als beim Stand der Technik, d.h. die Fläche 61 weiter in der Mitte der Prothese liegt, kann bei geringerer Steifigkeit des Kunststoffkörpers 1 die gleiche Steifigkeit der Prothese erreicht werden. Daher kann das Gewicht reduziert und somit der Tragekomfort der Prothese erhöht werden.

Zwischen der Fläche 61 und der innerhalb davon liegenden Rückseite 31 des Kunststoffkörpers 1 befindet sich ein Hohlraum 5. Wenn das Stoffteil 6 aus Microfaser- bzw. Klimastoffen gefertigt ist, kann in Dampfform durch das Stoffteil hindurchgetretener Schweiß dort kondensieren. Die der Trägerin zugewandete Seite bleibt angenehm trocken. Da der Hohlraum 5 sehr groß ausgebildet ist, ist das auch bei längerer Tragedauer der Fall.

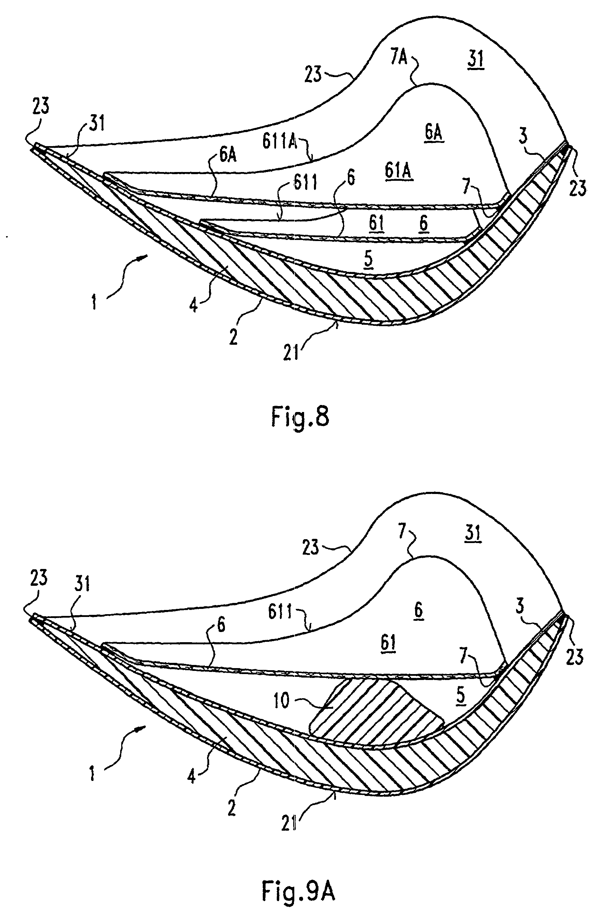

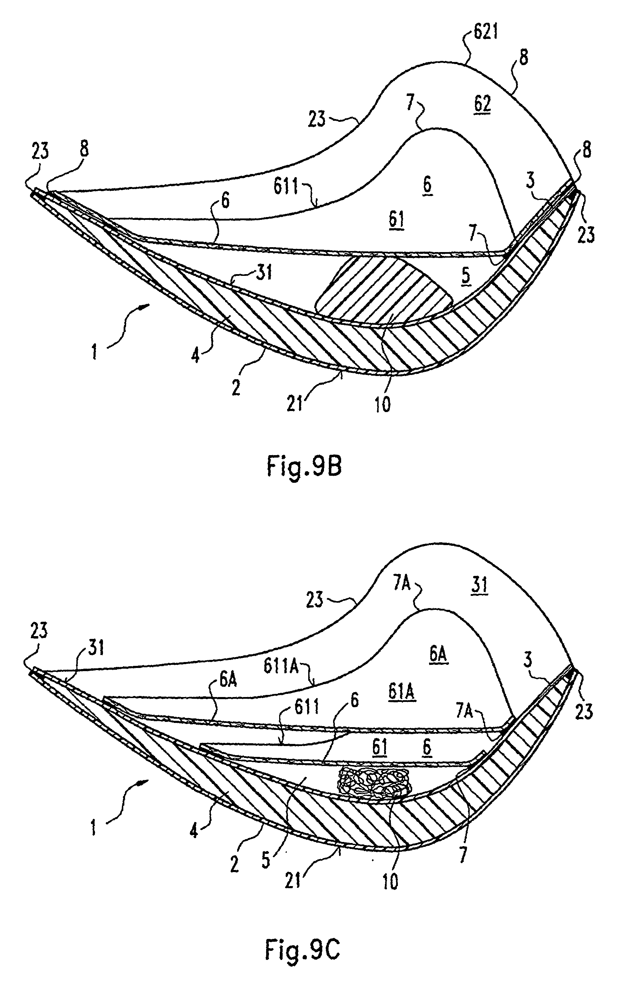

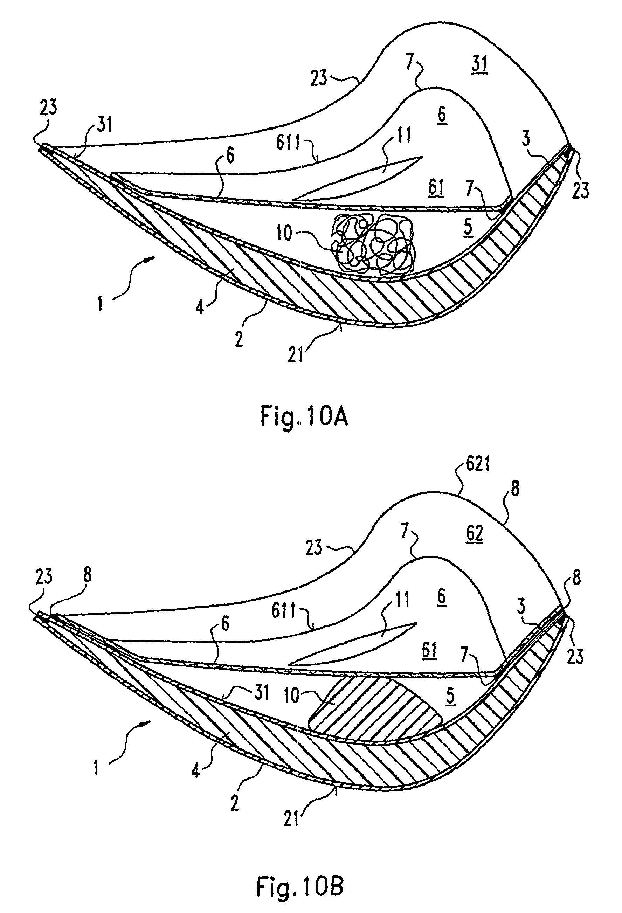

Anstelle einer festen Verbindung zwischen dem Stoffteil 6 und der Rückseite des Kunststoffkörpers 1 kann auch eine lösbare Verbindung in Betracht kommen. Eine lösbare Verbindung ist dann angebracht, wenn das Stoffteil 6 und der Kunststoffkörper 1 getrennt voneinander gereinigt werden sollen. Ein abnehmbares Stoffteil 6 ist auch dann von Vorteil, wenn der Hohlraum 5 wie bei den in den Figuren 9A, 9B, 9C, 10A und 10B dargestellten und unten beschriebenen Ausführungsbeispielen, einen Polsterkörper enthält, der bei Bedarf herausgenommen, gereinigt, ausgetauscht oder im Volumen verändert werden soll. Das Stoffteil 6 kann auch mit einer Öffnung versehen sein, durch die in den Hohlraum 5 eingegriffen werden kann, um für die vorgenannten Zwecke an den Polsterkörper zu gelangen oder um den Polsterkörper in den Hohlraum überhaupt einbringen zu können, wobei eine Öffnung insbesondere dann vorgesehen sein kann, wenn das Stoffteil fest mit der Rückseite des Kunststoffkörpers verbunden ist. Anstelle die Öffnung im Stoffteil vorzusehen, kann die Öffnung auch dadurch gebildet werden, daß die Verbindungsnaht zwischen Stoffteil und Kunststoffkörper unterbrochen wird. Eine lösbare Verbindung zwischen Stoffteil und Kunststoffkörper kann z.B. durch einen Klettverschluß realisiert werden.

In der folgenden Beschreibung weiterer Ausführungsbeispiele werden der Kürze halber Teile, die dieselben Bezugszeichen tragen und dieselbe Funktion erfüllen, nicht näher erläutert oder gar nicht erwähnt.

Bei dem in Figur 7 gezeigten, weiteren Ausführungsbeispiel der erfindungsgemäßen Brustprothese hat das Stoffteil 6 zusätzlich zu der zentralen Fläche 61 einen Randbereich 62, der mit der Rückseite 31 durch zwei Schweißnähte 7 und 8 fest verbunden ist. Dieser Randbereich 62 bedeckt auch den Bereich, an dem die Brustprothese auf der Haut der Prothesenträgerin aufliegt. Zusätzlich zu den Schweißnähten 7 und 8 ist er rasterförmig mit der Rückseite 31 fest verbunden. Er besteht vorzugsweise aus einem hautfreundlichen Material und insbesondere vorzugsweise aus einem Microfaserstoff, der Schweiß in der Dampfphase durchläßt, aber im flüssigen Zustand den Durchtritt versperrt. Deshalb bleibt der Kontaktbereich zwischen Brustprothese und Haut der Prothesenträgerin trocken und ein angenehmer Tragekomfort wird gewährleistet. Durch die rasterförmige Befestigung entstehen zusätzlich Luftkammern, die den Tragekomfort weiter verbessern. Die durch das Raster entstehende Verrippung dient zur Oberflächenvergrößerung der den Körper der Prothesenträgerin zugewandten Prothesenseite. Bei Verwendung von Microfaserstoff als Bedeckung für den Randbereich 62 kondensiert durchgehender Schweißdampf an der Kunststoffolie 3. Die Verrippung bewirkt einen sicheren und rutschfreien Sitz des Stoffes.

Figur 8 zeigt ein weiteres Ausführungsbeispiel der erfindungsgemäßen Brustprothese. Das Ausführungsbeispiel ist ähnlich zu dem in Figur 6 dargestellten. Es weist zusätzlich zu dem in der Figur 6 dargestelltem Ausführungsbeispiel ein weiteres flächiges Teil 6A mit einer Fläche 61A auf. Dadurch, daß die Flächen über einen weiten Bereich der Prothesenhöhe verteilt sind, kann bei gleicher Steifigkeit der Brustprothese die Steifigkeit des Kunststoffkörpers beispielsweise durch eine dünnere Bauweise weiter reduziert werden. Die damit einhergehende Gewichtsreduzierung steigert den Tragekomfort.

In einem alternativen nicht gezeigten Ausführungsbeispiel könnte auch das flächige Teil 6A weggelassen werden, so daß nur der vordere Bereich der Brustprothese durch das Stoffteil 6 versteift wird. Eine solche Brustprothese ist besonders für teilamputierte Frauen geeignet, bei denen ein großer Teil der Brust nicht entfernt wurde. Je nach Aufwand des Stützungsbedarfs können auch noch weitere flächige Teile 6A mit Flächen 61A mit dem Kunststoffkörper 1 fest oder lösbar verbunden werden.

Die Figuren 9A, 9B und 9C zeigen weitere Ausführungsbeispiele der erfindungsgemäßen Brustprothese, die denen der Figuren 6, 7 und 8 entsprechen. Es werden nur die Unterschiede zu den obigen Ausführungsbeispielen beschrieben. Für die Erläuterung bereits beschriebener Teile wird auf die obige Figurenbeschreibung verwiesen. Bei den Ausführungsbeispielen der Figuren 9A, 9B und 9C ist ein Polsterkörper 10 in dem Hohlraum 5 angeordnet. Dieser Polsterkörper 10 kann beispielsweise eine mit Luft gefüllte Polyurethanfolie oder ein Microfasergarnbausch oder ein Schaumstoffkörper sein. Durch den Polsterkörper entsteht eine zusätzliche Stabilisierung der Brustprothese ohne ihr Gewicht wesentlich zu erhöhen. Daher könnte der Silikonanteil bzw. der Raum zwischen den Kunststoffolien 2 und 3 noch weiter reduziert werden und die Prothese gewichtsreduzierend und kostensparend hergestellt werden. Dadurch wird der Tragekomfort weiter gesteigert.

Die Figuren 10A und 10B zeigen weitere Ausführungsbeispiele der erfindungsgemäßen Prothese, die im wesentlichen dem Ausführungsbeispiel der Figur 6 bzw. der Figur 7 entsprechen. Es werden nur die Unterschiede zu den obigen Ausführungsbeispielen beschrieben. Für die Erläuterung bereits beschriebener Teile wird auf die obige Figurenbeschreibung verwiesen. Das Ausführungsbeispiel von Figur 10A weist als Polsterkörper einen Microfaser-Garnbausch 10 auf. Dieser ist durch eine Öffnung 11, die in der Fläche 61 ausgebildet ist, entnehmbar oder ergänzbar. Dadurch kann die Steifigkeit bzw. Spannung der nachzubildenden menschlichen Brust angepaßt werden. Das Ausführungsbeispiel der Figur 10B weist als Polsterkörper einen Polyurethanball auf. Durch die Öffnung 11 kann weiteres Polstermaterial ergänzt werden bzw. der Polsterkörper entnommen werden. Dadurch ergeben sich die obigen Vorteile. Selbstverständlich können in beiden Ausführungsbeispielen als Polsterkörper beliebige Polstermaterialien verwendet werden. Das können beispielsweise auch ein Schaum oder Fiberfill sein. Auch ein mit Luft oder einem anderen Gas oder mit Flüssigkeit gefüllter Polsterkörper könnte verwendet werden. Ein derartiger Polsterkörper kann ein Ventil aufweisen, das eine Veränderung des Volumens und/oder der Härte des Polsterkörpers gestattet.

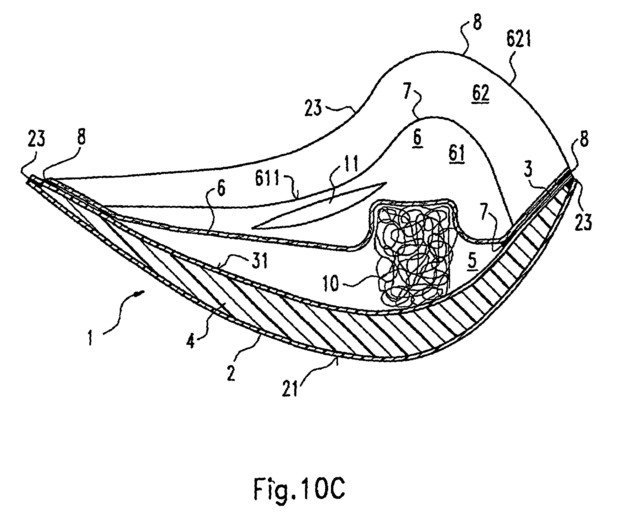

Die Verwendung eines Polsterkörpers, dessen Form und Volumen veränderbar ist, hat auch den Vorteil, daß die vom Stoffteil gebildete Fläche der Form des bei einer Teilamputation stehengebliebenen Gewebes individuell angepaßt werden kann. Ein Beispiel einer individuellen Anpassung der Fläche des Stoffteils durch eine entsprechende Formgebung des Polsterkörpers ist in Figur 10C gezeigt.

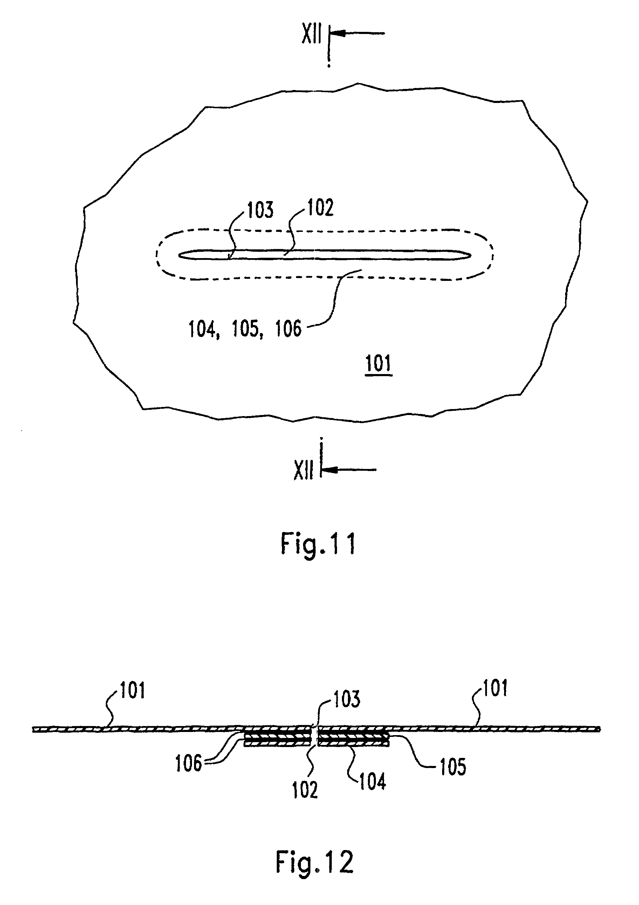

Die Figuren 11 und 12 zeigen eine vorteilhafte Ausgestaltung des Randes der Öffnung 102, die bei den anderen Figuren mit 11 bezeichnet ist, wobei der Rand dadurch verstärkt ist, daß eine Kunststoffolie 105 zwischen dem Stoffteil 101 (oben mit 6 bezeichnet) und einem Stoffband 104, das längs des Stoffrandes 103 angeordnet ist, angeordnet und mit dem Stoffteil 101 und dem Stoffband 104 verschweißt oder verklebt ist. Die Kunststoffolie ist vorzugsweise eine Polyurethanfolie und das Stoffteil 101 und das Stoffband 104 bestehen vorzugweise aus dem gleichen Material. Zu beachten ist, daß die Dicken der drei Teile übertrieben groß dargestellt sind und insbesondere die Kunststoffolie nach der Verschweißung in der Praxis kaum mehr gegenüber dem Stoffteil und dem Stoffband im Querschnitt in Erscheinung tritt.

Das Stoff teil 101 hat eine schlitzförmige Öffnung 102, dessen Rand 103 dadurch verstärkt ist, daß auf der Rückseite des Stoffteiles 101 ein ringförmiges Stoffband 104 so angeordnet ist, daß die die Öffnung 102 begrenzenden Kanten des Stoffteiles 101 und des Stoffbandes 104 bündig aufeinander liegen.

Wie aus Fig. 12 ersichtlich, befindet sich zwischen dem Stoffteil 101 und dem Stoffband 104 eine Kunststoffolie 105, welche den gleichen Umriß wie das Stoffband 104 hat und deckungsgleich mit dem Stoffband 104 angeordnet ist. Die Kunststoffolie 105 ist vollflächig mit dem Stoffteil 101 und dem Stoffband 104 verschweißt. Die Schweißverbindungen sind in Fig. 12 wie in den anderen Figuren durch dicke Striche 106 angedeutet.

In Figur 11 ist der Bereich des Stoffbandes 104, der Kunststoffolie 105 und der Schweißverbindungen durch die gestrichelte Linie angedeutet.

Die Schweißverbindung kann, wie in Fig. 12 gezeigt, so gestaltet sein, daß eine Verschmelzung nur an den Oberflächen der drei Teile stattfindet. Andererseits ist es auch möglich, die Schweißverbindung derart zu gestalten, daß alle drei Teile vollständig miteinander verschmelzen.

## Patentansprüche

1. Brustprothese mit
- einem weichelastischen Kunststoffkörper (1), der zwei längs ihres gemeinsamen Randes (23) miteinander verbundene Kunststoffolien (2, 3) und eine zwischen den Kunststoffolien (2, 3) eingeschlossene Kunststoffmasse (4) aufweist, wobei der Kunststoffkörper (1) eine der Form der natürlichen Brust nachgebildete Vorderseite (21) und eine dem Oberkörper der Person zuwendbare Rückseite (31) hat, und
- einem elastisch verformbaren, flächigen Teil (6), das an seinem Rand an der Rückseite (31) des Kunststoffkörpers (1) befestigt ist,
**dadurch gekennzeichnet, daß** das flächige Teil (6) 1 bis 4 cm innerhalb des gemeinsamen Randes (23) der miteinander verbundenen Kunststoffolien (2, 3) an dem Kunststoffkörper (1) befestigt ist.

2. Brustprothese nach Anspruch 1, **dadurch gekennzeichnet, daß** das flächige Teil (6) an seinem Rand mit der Rückseite (31) des Kunststoffkörpers (1) durch mindestens eine Schweißnaht (7) teilweise oder ganz verschweißt ist.

3. Brustprothese nach Anspruch 1, **dadurch gekennzeichnet, daß** das flächige Teil (6) an seinem Rand mit der Rückseite (31) verklebt ist.

4. Brustprothese nach einem der vorhergehenden Ansprüche, **dadurch gekennzeichnet, daß** das flächige Teil (6) nur an seinem Rand an der Rückseite (31) des Kunststoffkörpers (1) befestigt ist.

5. Brustprothese nach einem der vorhergehenden Ansprüche, **dadurch gekennzeichnet, daß** das flächige Teil (6) im ungespannten Zustand einen Flächeninhalt aufweist, der größer als die durch seinen an dem Kunststoffkörper (1) befestigten Rand definierte Minimalfläche ist.

6. Brustprothese nach einem der vorhergehenden Ansprüche,
**dadurch gekennzeichnet, daß** das flächige Teil (6) in ungespanntem Zustand einen Flächeninhalt aufweist, der im wesentlichen der von dem an dem Kunststoffkörper (1) befestigten Rand eingeschlossenen Fläche der Rückseite (31) des Kunststoffkörpers (1) entspricht.

7. Brustprothese nach einem der vorhergehenden Ansprüche, **dadurch gekennzeichnet, daß** das flächige Teil (6) ein Stoffteil ist.

8. Brustprothese nach einem der vorhergehenden Ansprüche, **dadurch gekennzeichnet, daß** das flächige Teil (6, 101) eine Öffnung (11, 102) zum Einbringen von Füllmaterial (10) zwischen das flächige Teil (6, 101) und die Rückseite (31) des Kunststoffkörpers (1) aufweist.

9. Brustprothese nach Anspruch 8, **dadurch gekennzeichnet, daß** die Öffnung (11, 102) schlitzfömig ist, und das flächige Teil (6, 101) an dem Rand der Öffnung (11, 102) durch ein Stoffband (104) verstärkt ist.

10. Brustprothese nach Anspruch 9, **dadurch gekennzeichnet, daß** eine Kunststoffolie (105) zwischen dem flächigen Teil (6, 101) und dem Stoffband (104) angeordnet ist und mit dem flächigen Teil (6, 101) und dem Stoffband (104) verschweißt oder verklebt ist.

11. Brustprothese nach Anspruch 9 oder 10, **dadurch gekennzeichnet, daß** das flächige Teil (6, 101) und das Stoffband (104) aus dem gleichen Material bestehen.

12. Brustprothese nach einem der vorhergehenden Ansprüche, **dadurch gekennzeichnet, daß** die Kunststoffolien (2, 3, 105) Polyurethanfolien sind.

13. Brustprothese nach einem der vorhergehenden Ansprüche, **dadurch gekennzeichnet, daß** das flächige Teil (6) mit dem Kunststoffkörper (1) einen Hohlraum (5) einschließt.

14. Brustprothese nach einem der vorhergehenden Ansprüche, **dadurch gekennzeichnet, daß** der Rand (611) des flächigen Teils (6) im vorderen Drittel des Kunststoffkörpers (1) angeordnet ist.

15. Brustprothese nach einem der vorhergehenden Ansprüche, **dadurch gekennzeichnet, daß** das flächige Teil (6) einen Randbereich (62) aufweist, der eine zentrale Fläche (61) umgibt und sich bis zu dem gemeinsamen Rand (23) der beiden Kunststoffolien (2, 3) erstreckt.

16. Brustprothese nach Anspruch 15, **dadurch gekennzeichnet, daß** der Randbereich (62) einen mit dem gemeinsamen Rand (23) der Kunststoffolien (2, 3) abschließenden Rand (621) aufweist, und der abschließende Rand (621) des Randbereichs (62) mit dem gemeinsamen Rand (23) der Kunststoffolien fest verbunden ist.

17. Brustprothese nach Anspruch 15 oder 16, **dadurch gekennzeichnet, daß** der abschließende Rand (621) mit dem Kunststoffkörper (1) durch eine zweite Schweißnaht (8) verschweißt ist.

18. Brustprothese nach Anspruch 15 oder 16, **dadurch gekennzeichnet, daß** der Randbereich (62) an dem abschließenden Rand (621) mit dem Kunststoffkörper (1) verklebt ist.

19. Brustprothese nach einem der Ansprüche 15 bis 18, **dadurch gekennzeichnet, daß** der Rand (62) rasterähnlich mit der Rückseite (31) des Kunststoffkörpers (1) fest verbunden ist.

20. Brustprothese nach einem der vorhergehenden Ansprüche, **dadurch gekennzeichnet, daß** die Brustprothese mindestens ein weiteres flächiges Teil (6A) aufweist, das an seinem Rand derart an der Rückseite (31) des Kunststoffkörpers (1) befestigt ist, daß zwischen dem einen flächigen Teil (6) und dem weiteren flächigen Teil (6A) ein Abstand ist.

21. Brustprothese nach Anspruch 20, **dadurch gekennzeichnet, daß** der Rand des weiteren flächigen Teils (6A) in dem vorderen Drittel des Kunststoffkörpers (1) angeordnet ist, und der Rand (611) des einen flächigen Teils (6) in dem hinteren Drittel des Kunststoffkörpers (1) angeordnet ist.

22. Brustprothese nach einem der Ansprüche 8 bis 21, **dadurch gekennzeichnet, daß** die Öffnung (11) durch eine Unterbrechung der Schweißnaht oder Klebenaht (7) gebildet ist.

23. Brustprothese nach einem der Ansprüche 8 bis 22, **dadurch gekennzeichnet, daß** die Öffnung (11, 102) verschließbar ist.

24. Brustprothese nach einem der vorhergehenden Ansprüche, **dadurch gekennzeichnet, daß** in dem Hohlraum (5) ein Polsterkörper (10) angeordnet ist.

25. Brustprothese nach einem der vorhergehenden Ansprüche, **dadurch gekennzeichnet, daß** das oder mindestens ein flächiges Teil (6; 6A) ein Stoffteil ist.

26. Brustprothese nach Anspruch 25, **dadurch gekennzeichnet, daß** das Stoffteil (6, 6A) Polyamid- und/oder Polyesterfasern und/oder Baumwolle aufweist.

27. Brustprothese nach Anspruch 25, **dadurch gekennzeichnet, daß** das Stoffteil (6, 6A) aus einem Gewebe oder Gewirk aus Microfasern besteht.

28. Brustprothese nach Anspruch 27, **dadurch gekennzeichnet, daß** die Microfasern aus Polyester oder Polyamid bestehen.

29. Brustprothese nach einem der vorhergehenden Ansprüche, **dadurch gekennzeichnet, daß** das oder mindestens ein flächiges Teil (6; 6A) eine Kunststoffolie ist.

30. Brustprothese nach Anspruch 29, **dadurch gekennzeichnet, daß** das flächige Teil (6) eine Kunststoffolie ist und das weitere flächige Teil (6A) ein Stoffteil ist.

31. Verfahren zur Herstellung einer Brustprothese mit folgenden Schritten:
(A) Verbinden zweier Kunststoffolien (2, 3);
(B) Einbringen einer Kunststoffmasse zwischen die beiden Kunststoffolien (2, 3);
(C) Tiefziehen der Kunststoffolien (2, 3) zur Nachbildung der natürlichen Brust; und
(D) Vernetzen der Kunststoffmasse (4),
**dadurch gekennzeichnet, daß** vor dem Verbinden der beiden Kunststoffolien (2, 3) ein flächiges Teil (6, 6A) mit einer der beiden Kunststoffolien (3) 1 bis 4 cm innerhalb der Linie (23) verbunden wird, an der die beiden Kunststoffolien (2, 3) miteinander verbunden werden.

32. Verfahren nach Anspruch 31, **dadurch gekennzeichnet, daß** in das flächige Teil (6, 6A, 101) vor der Verbindung mit der einen Kunststoffolie (3) eine Öffnung (11, 102) gemacht wird.

33. Verfahren nach Anspruch 32, **dadurch gekennzeichnet, daß** eine Öffnung (11, 102) vor der Verbindung des flächigen Teils (6, 6A, 101) mit der einen Kunststoffolie (3) durch ein Stoffband (104) verstärkt wird, das mit dem flächigen Teil (6, 6A, 101) verbunden wird.

34. Verfahren nach Anspruch 33, **dadurch gekennzeichnet, daß** das flächige Teil (6, 6A, 101) aus Stoff ist, und zwischen das Stoffband (104) und das flächige Teil (6, 6A, 101) eine Kunststoffolie (105) geschweißt wird.

## Claims

1. A breast prosthesis comprising
• a soft-elastic plastic member (1) having two plastic sheets (2,3) interconnected along their common edge (23), and a plastic material (4) enclosed between the plastic sheets (2, 3), the plastic member (1) having a front (21) which simulates the shape of the natural breast and a back (31) adapted to face the wearer's upper body, and
• an elastically deformable flat part (6) which at its edge is secured to the back (31) of the plastic member (1) outside the plastic sheets,
**characterised in that** the flat part (6) is fixed to the plastic member (1) 1 to 4 cm inside the common edge (23) of the interconnected plastic sheets (2, 3).

2. A breast prosthesis according to claim 1, **characterised in that** the flat part (6) is partially or completely welded at its edge to the back (31) of the plastic member (1) by at least one weld seam (7).

3. A breast prosthesis according to claim 1, **characterised in that** the flat part (6) is stuck at its edge to the back (31).

4. A breast prosthesis according to any one of the preceding claims, **characterised in that** the flat part (6) is fixed only at its edge to the back (31) of the plastic member (1).

5. A breast prosthesis according to any one of the preceding claims, **characterised in that** the flat part (6) in the untensioned state has a surface area larger than the minimum area defined by its edge fixed to the plastic member (1).

6. A breast prosthesis according to any one of the preceding claims, **characterised in that** the flat part (6) in the untensioned state has a surface area which corresponds substantially to that area of the back (31) of the plastic member (1) which is enclosed by the edge fixed on the plastic member (1).

7. A breast prosthesis according to any one of the preceding claims, **characterised in that** the flat part (6) is a fabric part.

8. A breast prosthesis according to any one of the preceding claims, **characterised in that** the flat part (6, 101) has an opening (11, 102) for the introduction of filling material (10) between the flat part (6, 101) and the back (31) of the plastic member (1).

9. A breast prosthesis according to claim 8, **characterised in that** the opening (11,102) is in the form of a slot, and the flat part (6, 101) is reinforced by a fabric band (104) at the edge of the opening (11, 102).

10. A breast prosthesis according to claim 9, **characterised in that** a plastic sheet (105) is disposed between the flat part (6, 101) and the fabric band (104) and is welded or glued to the flat part (6, 101) and the fabric band (104).

11. A breast prosthesis according to claim 9 or 10, **characterised in that** the flat part (6, 101) and the fabric band (104) consist of the same material.

12. A breast prosthesis according to any one of the preceding claims, **characterised in that** the plastic sheets (2, 3, 105) are polyurethane sheets.

13. A breast prosthesis according to any one of the preceding claims, **characterised in that** the flat part (6) encloses a cavity (5) with the plastic member (1).

14. A breast prosthesis according to any one of the preceding claims, **characterised in that** the edge (611) of the flat part (6) is disposed in the front third of the plastic member (1).

15. A breast prosthesis according to any one of the preceding claims, **characterised in that** the flat part (6) has an edge zone (62) which surrounds a central surface (61) and which extends as far as the common edge (23) of the two plastic sheets (2, 3).

16. A breast prosthesis according to claim 15, **characterised in that** the edge zone (62) has an edge (621) flush with the common edge (23) of the plastic sheets (2, 3) and the flush edge (621) of the edge zone (62) is rigidly connected to the common edge (23) of the plastic sheets.

17. A breast prosthesis according to claim 15 or 16, **characterised in that** the flush edge (621) is welded to the plastic member (1) by a second weld seam (8).

18. A breast prosthesis according to claim 15 or 16, **characterised in that** the edge zone (62) is glued to the plastic member (1) at the flush edge (621).

19. A breast prosthesis according to any one of claims 15 to 18, **characterised in that** the edge (62) is rigidly connected to the back (31) of the plastic member (1) after the style of a grid.

20. A breast prosthesis according to any one of the preceding claims, **characterised in that** the breast prosthesis has at least one other flat part (6A) which at its edge is so fixed to the back (31) of the plastic member (1) that there is a space between the one flat part (6) and the other flat part (6A).

21. A breast prosthesis according to claim 20, **characterised in that** the edge of the other flat part (6A) is disposed in the front third of the plastic member (1) and the edge (611) of the one flat part (6) is disposed in the rear third of the plastic member (1).

22. A breast prosthesis according to any one of claims 8 to 21, **characterised in that** the opening (11) is formed by a break in the weld seam or glue seam (7).

23. A breast prosthesis according to any one of claims 8 to 22, **characterised in that** the opening (11, 102) is closable.

24. A breast prosthesis according to any one of the preceding claims, **characterised in that** a padding member (10) is disposed in the cavity (5).

25. A breast prosthesis according to any one of the preceding claims, **characterised in that** the or at least one flat part (6, 6A) is a fabric part.

26. A breast prosthesis according to claim 25, **characterised in that** the fabric part (6, 6A) comprises polyamide and/or polyester fibres and/or cotton.

27. A breast prosthesis according to claim 25, **characterised in that** the fabric part (6, 6A) consists of a woven or knitted fabric of microfibres.

28. A breast prosthesis according to claim 27, **characterised in that** the microfibres consist of polyester or polyamide.

29. A breast prosthesis according to any one of the preceding claims, **characterised in that** the or at least one flat part (6, 6A) is a plastic sheet.

30. A breast prosthesis according to claim 29, **characterised in that** the flat part (6) is a plastic sheet and the other flat part (6A) is a fabric part.

31. A method of producing a breast prosthesis according to claim 1, comprising the following steps:
(A) connecting two plastic sheets (2, 3);
(B) introducing a plastic material between the two plastic sheets (2, 3);
(C) deep-drawing the plastic sheets (2, 3) to simulate the natural breast; and
(D) cross-linking the plastic material (4),
**characterised in that** before the two plastic sheets (2, 3) are connected a flat part (6, 6A) is connected to one of the two plastic sheets (3) 1 to 4 cm within the line (23) at which the two plastic sheets (2, 3) are interconnected.

32. A method according to claim 31, **characterised in that** an opening (11, 102) is made in the flat part (6, 6A, 101) before connection to the one plastic sheet (3).

33. A method according to claim 32, **characterised in that** before the connection of the flat part (6, 6A, 101) to the one plastic sheet (3) an opening (11, 102) is reinforced by a fabric band (104) which is connected to the flat part (6, 6A, 101).

34. A method according to claim 33, **characterised in that** the flat part (6, 6A 101) is a fabric and a plastic sheet (105) is welded between the fabric band (104) and the flat part (6, 6A, 101).

## Revendications

1. Prothèse du sein, comportant
- un corps élastique souple en matière plastique (1), qui présente deux feuilles (2, 3) en matière plastique reliées l'une à l'autre le long de leur bord (23) commun, et une masse (4) en matière plastique enfermée entre les feuilles (2, 3) en matière plastique, le corps (1) en matière plastique présentant une face frontale (21) imitant la forme du sein naturel et une face dorsale (31) à appliquer sur le torse de la personne, et
- une partie mince (6), élastique, déformable, qui est fixée à son bord à la face dorsale (31) du corps (1) en - matière plastique,
**caractérisée en ce que** la partie mince (6) est fixée au corps (1) en matière plastique entre 1 et 4 cm à l'intérieur du bord (23) commun aux feuilles (2, 3) en matière plastique reliées l'une à l'autre.

2. Prothèse du sein selon la revendication 1, **caractérisée en ce que** la partie mince (6) est soudée, partiellement ou totalement à son bord, à la face dorsale (31) du corps (1) en matière plastique, par au moins un cordon de soudure (7).

3. Prothèse du sein selon la revendication 1, **caractérisée en ce que** la partie mince (6) est collée à son bord à la face dorsale (31).

4. Prothèse du sein selon l'une des revendications précédentes, **caractérisée en ce que** la partie (6) mince n'est fixée qu'à son bord, à la face dorsale (31) du corps en matière plastique (1).

5. Prothèse du sein selon l'une des revendications précédentes, **caractérisée en ce que** la partie mince (6) présente à l'état non tendu une superficie supérieure à sa surface minimale définie à son bord fixé au corps (1) en matière plastique.

6. Prothèse du sein selon l'une des revendications précédentes, **caractérisée en ce que** la partie mince (6) présente à l'état non tendu une superficie qui correspond essentiellement à la surface de la face dorsale (31) du corps (1) en matière plastique, qui est entourée par le bord fixé au corps (1) en matière plastique.

7. Prothèse du sein selon l'une des revendications précédentes, **caractérisée en ce que** la partie mince (6) est une pièce de tissu.

8. Prothèse du sein selon l'une des revendications précédentes, **caractérisée en ce que** la partie mince (6, 101) présente une ouverture (11, 102) pour l'insertion de matériau de remplissage (10) entre la partie mince (6, 101) et la face dorsale (31) du corps (1) en matière plastique.

9. Prothèse du sein selon la revendication 8, **caractérisée en ce que** l'ouverture (11, 102) présente la forme d'une fente, et **en ce que** la partie mince (6, 101) est renforcée par une bande de tissu (104) au bord de l'ouverture (11, 102).

10. Prothèse du sein selon la revendication 9, **caractérisée en ce qu'**une feuille (105) en matière plastique est disposée entre la partie mince (6, 101) et la bande de tissu (104) et est soudée ou collée à la partie mince (6, 101) et à la bande de tissu (104).

11. Prothèse du sein selon la revendication 9 ou 10, **caractérisée en ce que** la partie mince (6, 101) et la bande de tissu (104) sont constituées du même matériau.

12. Prothèse du sein selon l'une des revendications précédentes, **caractérisée en ce que** les feuilles en matière plastique (2, 3, 105) sont des feuilles de polyuréthane.

13. Prothèse du sein selon l'une des revendications précédentes, **caractérisée en ce que** la partie mince (6) délimite, avec le corps en matière plastique (1), un espace creux (5).

14. Prothèse du sein selon l'une des revendications précédentes, **caractérisée en ce que** le bord (611) de la partie mince (6) est disposé dans le tiers frontal du corps (1) en matière plastique.

15. Prothèse du sein selon l'une des revendications précédentes, **caractérisée en ce que** la partie mince (6) présente une région de bordure (62) qui entoure une surface centrale (61) et s'étend jusqu'au bord (23) commun aux deux feuilles en matière plastique (2, 3).

16. Prothèse du sein selon la revendication 15, **caractérisée en ce que** la région de bordure (62) présente un bord de séparation (621) avec le bord commun (23) des feuilles en matière plastique (2, 3), et **en ce que** le bord de séparation (621) de la région de bordure (62) est lié fermement au bord commun (23) des feuilles de matière plastique.

17. Prothèse du sein selon les revendications 15 ou 16, **caractérisée en ce que** le bord de séparation (621) est soudé au corps (1) en matière plastique par un deuxième cordon de soudure (8).

18. Prothèse du sein selon la revendication 15 ou 16, **caractérisée en ce que** la région de bordure (62) est collée au corps (1) en matière plastique par le bord de séparation (621).

19. Prothèse du sein selon l'une des revendications 15 à 18, **caractérisée en ce que** le bord (62) est lié fermement à la face dorsale (31) du corps (1) en matière plastique à la manière d'une grille.

20. Prothèse du sein selon l'une des revendications précédentes, **caractérisée en ce que** la prothèse du sein présente au moins une autre partie mince (6A), qui est fixée à son bord à la face dorsale (31) du corps (1) en matière plastique de manière à ce qu'il existe un écart entre l'une des parties minces (6) et l'autre partie mince (6A).

21. Prothèse du sein selon la revendication 20, **caractérisée en ce que** le bord de l'autre partie mince (6A) est disposé dans le tiers frontal du corps (1) en matière plastique, et **en ce que** le bord (611) de la première partie mince (6) est disposé dans le tiers arrière du corps (1) en matière plastique.

22. Prothèse du sein selon l'une des revendications 8 à 21, **caractérisées en ce que** l'ouverture (11) est formée par une interruption du cordon de soudure ou du cordon de colle (7).

23. Prothèse du sein selon l'une des revendications 8 à 22, **caractérisée en ce que** l'ouverture (11, 102) est refermable.

24. Prothèse du sein selon l'une des revendications précédentes, **caractérisée en ce qu'**un corps de rembourrage (10) est disposé dans l'espace creux (5).

25. Prothèse du sein selon l'une des revendications précédentes, **caractérisée en ce que** la partie mince ou au moins l'une des parties minces (6; 6A) est une partie en tissu.

26. Prothèse du sein selon la revendication 25, **caractérisée en ce que** la partie en tissu (6, 6A) présente des fibres de polyamide et/ou de polyester, et/ou de coton.

27. Prothèse du sein selon la revendication 25, **caractérisée en ce que** la pièce en tissu (6, 6A) est constituée d'un tissu ou d'un tricot en microfibres.

28. Prothèse du sein selon la revendication 27, **caractérisée en ce que** les microfibres sont constituées de polyester ou de polyamide.

29. Prothèse du soin selon l'une des revendications précédentes, **caractérisée en ce que** la partie mince, ou au moins l'une des parties minces (6; 6A) est une feuille en matière plastique.

30. Prothèse du sein selon la revendication 29 **caractérisée en ce que** la partie mince (6) est une feuille en matière plastique et l'autre partie mince (6A) est une partie en tissu.

31. Procédé pour la fabrication d'une prothèse, comportant les étapes consistant à:
(A) lier deux feuilles en matière plastique (2, 3);
(B) introduire une masse en matière plastique entre les deux feuilles en matière plastique (2, 3);
(C) emboutir les feuilles en matière plastique (2, 3) pour imiter le sein naturel; et
(D) réticuler la masse de matière plastique (4),
**caractérisé en ce que**, avant la liaison des deux feuilles en matière plastique (2, 3), une partie mince (6, 6A) est liée à l'une (3) des deux feuilles en matière plastique entre 1 et 4 cm à l'intérieur de la ligne (7) suivant laquelle les deux feuilles en matière plastique (2, 3) sont reliées l'une à l'autre.

32. Procédé selon la revendication 31, **caractérisée en ce que**, avant la liaison à la première feuille en matière plastique (3), une ouverture (11, 102) est réalisée dans la partie mince (6, 6A, 101).

33. Procédé selon la revendication 32, **caractérisé en ce que**, avant la liaison de la partie mince (6, 6A, 101) à la première feuille (3) en matière plastique, une ouverture (11, 102) est renforcée par une bande de tissu (104) qui est reliée à la partie mince (6, 6A, 101).

34. Procédé selon la revendication 33, **caractérisée en ce que** la partie mince (6, 6A, 101) est en tissu, et **en ce que** qu'une feuille (105) en matière plastique est soudée entre la bande de tissu (104) et la partie mince (6, 6A, 101).
